# EUROPEAN PATENT SPECIFICATION

(11) **EP 3 154 440 B1**
(45) Date of publication and mention of the grant of the patent: **04.12.2019**
(21) Application number: 15726151.2
(22) Date of filing: 02.06.2015
(51) Int. Cl.: A61B 10/02, A61B 17/3207, A61B 90/00, A61B 17/00

(54) **ENDOSCOPIC BIOPSY INSTRUMENT**
ENDOSKOPISCHES BIOPSIEINSTRUMENT
INSTRUMENT DE BIOPSIE ENDOSCOPIQUE

(30) Priority: 10.06.2014 SE 1450711
(43) Date of publication of application: 19.04.2017
(73) Proprietor: BibbInstruments AB, 223 63 Lund (SE)
(72) Inventor: WALTHER Charles, 222 23 Lund (SE); DYMLING Stephan, 216 22 Limhamn (SE); WALTHER Bruno, 240 33 Löberöd (SE)
(74) Representative: AWA Sweden AB
(86) International application number: PCT/EP2015/062210
(87) International publication number: WO 2015/189067

(56) References cited:
- WO-A1-00/13595
- WO-A1-2013/179013
- DE-U1- 9 312 792
- US-A1- 2004 162 565
- US-A1- 2010 312 141
- US-A1- 2013 223 702
- US-A1- 2014 155 905

## Description

### Technical field

The present invention relates to the field of endoscopic biopsy.

### Background

A biopsy is a medical test commonly performed by a surgeon involving sampling of cells or tissues for examination. A large variety of endoscopic biopsy instruments are commercially available today, the majority of which are biopsy forceps that operate by the forceps being closed to pinch of the tissue sample.

For some diagnostic purposes, the millimeter-sized samples retrievable using forceps are sufficient, but for some types of lesions and tumors, such a small and superficial sample is inadequate for making a diagnosis. An endoscopic biopsy instrument for obtaining a deeper tissue sample from a patient is for example disclosed in PCT publication WO2014020150 (to Walther).

When taking a tissue sample with an endoscopic biopsy instrument, the instrument is inserted in a working channel of an endoscope, and the forceps are advanced to the biopsy site. The forceps are usually operated by means of a syringe-type handle, which usually offers sufficient precision for operating a forceps.

However, in many applications there is a need for obtaining tissue at a more specific location and at a specific depth. It would therefore be advantageous to have an improved way of controlling the location and depth of the position in which a tissue sample is obtained.

US Patent Application publication No. 2010/0312141 A1 (to Keast) shows a biopsy instrument for use in the airways through the working channel of a bronchoscope.

### Summary

The invention relates to an endoscopic biopsy instrument according to claim 1.

An endoscopic biopsy instrument for obtaining a tissue sample from a body of a patient is provided, The endoscopic biopsy instrument comprises a proximal portion adapted to remain outside of the body of the patient in use, and a distal portion adapted to be inserted into the body of the patient in use. The endoscopic biopsy instrument further comprises a sleeve extending from the proximal portion to the distal portion, and a motion transferring member extending from the proximal portion to the distal portion within the sleeve. The motion transferring member is configured to transfer rotary and linear motion.

The distal portion of the endoscopic biopsy instrument comprises a distal end of the motion transferring member, adapted to be connected to a rotatable cutting tip being extendable from a distal end of the sleeve. The rotatable cutting tip is configured to obtain a tissue sample from within the body of the patient using rotary motion.

The proximal portion of the endoscopic biopsy instrument comprises a handle portion connected to the sleeve. The handle portion comprises a rotary motion element connected to the motion transferring member for transferring rotary motion to the rotatable cutting tip, and a linear motion element connected to the cutting tip via the motion transferring member, for extending and retracting the cutting tip from the distal portion of the sleeve. The proximal portion further comprises a lockable adjustment device for, in situ, adjusting and locking a maximum length that the rotatable cutting tip extends from the distal portion of the sleeve, when being extended and retracted by the linear motion element. The lockable adjustment device enables the depth of the tissue sample to be obtained to be controlled, in situ, which increases the precision of the depth control.

According to one embodiment, the linear motion element has an innermost position, in which the cutting tip extends maximally from the distal end of the sleeve. The lockable adjustment device may be adapted to adjust the distance between a proximal end of the sleeve and the innermost position, for adjusting the maximum length that the rotatable cutting tip extends from the distal portion of the sleeve when being extended and retracted by the linear motion element.

According to one embodiment, the lockable adjustment device comprises an adjustment sleeve being axially displaceable in relation to the sleeve, and the lockable adjustment device further comprises a locking device for axially locking the adjustment sleeve in relation to the sleeve and thereby adjusting and locking the maximum length that the rotatable cutting tip extends from the sleeve. In one embodiment, a proximal portion of the sleeve comprises a threaded portion, and the adjustment sleeve comprises threads being adapted to engage the threads of the sleeve, such that the adjustment sleeve can be axially displaced in relation to the sleeve by the adjustment sleeve and the sleeve being radially rotated in relation to each other. The locking device may comprise a threaded locking member adapted to engage the threads of at least one of the sleeve and the adjustment sleeve, for locking the sleeve in relation to the adjustment sleeve. The embodiments with threads enable precise adjustment of the adjustment sleeve and locking member, whilst making sure that the adjustable locking member can withstand exerted forces.

In an alternative embodiment, the lockable adjustment device comprises a locking device comprising a radially displaceable part adapted to engage at least one of the motion transferring member, the sleeve and the adjustment sleeve. The radially displaceable part is thereby adapted to lock the maximum length that the rotatable cutting tip extends from the sleeve, when being extended and retracted by the linear motion element. The adjustment sleeve may be a compressible adjustment sleeve comprising the radially displaceable part, such that compression of the compressible adjustment sleeve disengages the radially displaceable part from the motion transferring member, the sleeve or the adjustment sleeve, such that the maximum length that the rotatable cutting tip extends from the distal portion of the sleeve can be adjusted. The release of the compression of the compressible adjustment sleeve engages the radially displaceable part to the motion transferring member the sleeve or the adjustment sleeve, such that the lockable adjustment device is locked. The radially displaceable part or the compressible adjustment sleeve may comprise a flexible or elastic polymer material.

The rotary motion element and the linear motion element may be a combined element, such that rotary and linear motion can be generated single-handedly.

The endoscopic biopsy instrument according to any one of the preceding embodiments may be configured for stepless operation of at least one of: the adjustment of the maximum length that the rotatable cutting tip extends from the sleeve, the extension and retraction of the rotatable cutting tip, and the rotary motion of the cutting tip.

According to one embodiment the sleeve is a flexible sleeve and/or the motion transferring member is a flexible motion transferring member, such that the endoscopic biopsy instrument can be inserted through a flexible endoscope.

The rotatable cutting tip may comprise a drill, and the flexible motion transferring member may be a flexible shaft.

According to one embodiment, the endoscopic biopsy instrument or the motion transferring member may have a length exceeding one of 10cm, 20cm, 30cm, 40cm, 50cm, 60cm, 70cm, 80cm, 90cm or 100cm e.g. for being able to reach areas of the patient's digestive system or airways.

The flexible sleeve may comprise a helically wound metal sleeve and/or a flexible sleeve made from a polymer material, and/or a woven or braided flexible metal sleeve.

According to one embodiment, the linear motion element extends partially inside of the adjustment sleeve and the adjustment sleeve extends partially inside of the sleeve, creating a stable elongated handle portion.

The endoscopic biopsy instrument according to any one of the preceding embodiments may comprise grip improving protrusions or recesses on at least one of: the rotary motion element, the linear motion element and the lockable adjustment device. The grip improving protrusions or recesses may be axially extending grooves.

A method of obtaining a tissue sample from a body of a patient is further provided. The method uses an endoscopic biopsy instrument comprising a sleeve and a rotatable cutting tip operated from a handle portion of the endoscopic biopsy instrument. The cutting tip is configured to obtain a tissue sample from within the body of the patient using rotary motion. The method comprises inserting the endoscopic biopsy instrument through an endoscope into the body of the patient, and adjusting a maximum length that the rotatable cutting tip extends from the sleeve, when being extended and retracted by a linear motion element of the handle portion. The method further comprises using a lockable adjustment device of the endoscopic biopsy instrument for locking the lockable adjustment device, and rotating, by means of the handle portion, the rotatable cutting tip for obtaining a tissue sample. After the tissue sample has been obtained, the rotatable cutting tip is retracted into the sleeve using the linear motion element, and the endoscopic biopsy instrument is retracted from the body of the patient.

The method may be preceded by the steps of placing the endoscope such that it extends from a location outside the body of the patient to a location inside the body of the patient, and the step of inflating an area of the patient with a gas for creating a cavity within the body of the patient allowing visual inspection.

The endoscope may comprise a camera for visual inspection, such that the step of adjusting a maximum length that the rotatable cutting tip extends from the sleeve can be performed under visual inspection.

The endoscope may be a gastroscope, and the step of inserting the endoscopic biopsy instrument may comprise inserting the endoscopic biopsy instrument through the gastroscope into the digestive system of the patient, and the step of obtaining a tissue sample may comprise obtaining a tissue sample from the digestive system of the patient.

Alternatively, the endoscope may be a colonoscope, and the step of inserting the endoscopic biopsy instrument through an endoscope into the body of the patient may comprise inserting the endoscopic biopsy instrument through the colonoscope into the digestive system of the patient, and the step of obtaining a tissue sample may comprise obtaining a tissue sample from the digestive system of the patient.

Alternatively, the endoscope may be a bronchoscope, and the step of inserting the endoscopic biopsy instrument through an endoscope into the body of the patient may comprise inserting the endoscopic biopsy instrument through the bronchoscope into the airways of the patient, and the step of obtaining a tissue sample may comprise obtaining a tissue sample from the airways of the patient.

Alternatively, the endoscope may be a rhinoscope, and the step of inserting the endoscopic biopsy instrument through an endoscope into the body of the patient may comprise inserting the endoscopic biopsy instrument through the rhinoscope into the nose of the patient, and the step of obtaining a tissue sample may comprise obtaining a tissue sample from the nose of the patient.

The different aspects or part of the aspects of the disclosed embodiments may all be combined in any possible way, unless clearly contradictory. Any method or any step of a method should be seen also as a description of the elements necessary to carry out said method.

### Brief description of drawings

The invention is now described, by way of example, with reference to the accompanying drawings, on which:
Fig. 1 shows an embodiment of the endoscopic biopsy instrument in an exploded elevated view.
Fig. 2 shows a detailed sectional view of the handle portion of the endoscopic biopsy instrument.
Fig. 3 is a flow chart of a method of using the endoscopic biopsy instrument.
Fig. 4A shows a side view of the proximal portion of the endoscopic biopsy instrument and a sectional view of the distal portion of the endoscopic biopsy instrument when a tissue sample is being obtained.
Fig. 4B shows a side view of the proximal portion of the endoscopic biopsy instrument and a sectional view of the distal portion of the endoscopic biopsy instrument when the tissue sample has been retracted into the sleeve of the distal portion.

### Detailed description

In the following, a detailed description of embodiments of the invention will be given with reference to the accompanying drawings. It will be appreciated that the drawings are for illustration only and are not in any way restricting the scope of the invention. Thus, any references to directions, such as "up" or "down", are only referring to the directions shown in the figures.

An endoscopic biopsy instrument for obtaining a tissue sample from the body of the patient is provided. The endoscopic biopsy instrument comprises a proximal portion adapted to remain outside of the body of the patient, when the endoscopic biopsy instrument is in use, and a distal portion adapted to be inserted into the body of the patient when the endoscopic biopsy instrument is in use. The endoscopic biopsy instrument further comprises a sleeve extending from the proximal portion to the distal portion, which may be a rigid or flexible sleeve, and a motion transferring member extending from the proximal portion to the distal portion within the sleeve. The motion transferring member may be a rigid or flexible motion transferring member depending on the specific application of the endoscopic biopsy instrument.

The distal portion comprises a distal end of the motion transferring member adapted to be connected to a rotatable cutting tip being extendable from a distal end of the sleeve, and being configured to obtain a tissue sample from within the body of the patient using rotary motion. The motion transferring member is configured to transfer rotary and linear motion, i.e. the motion transferring member is configured to transfer motion for extracting/retracting and rotating the rotatable cutting tip.

The proximal portion of the endoscopic biopsy instrument comprises a handle portion connected to the sleeve. The handle portion comprises a rotary motion element connected to the motion transferring member for transferring rotary motion to the rotatable cutting tip, and a linear motion element connected to the cutting tip via the motion transferring member, for extending and retracting the cutting tip from the distal portion of the sleeve. The handle portion further comprises a lockable adjustment device for, in situ, adjusting and locking a maximum length that the rotatable cutting tip extends from the distal portion of the sleeve, when being extended and retracted by the linear motion element. The handle portion enables the adjustment to take place after the endoscopic biopsy instrument has been inserted into the body of the patient and thus enables the adjustment to be performed in the position in which the tissue of the patient is obtained.

In one embodiment, the sleeve (or a portion of the sleeve) is an elongated sleeve which is flexible, such that the endoscopic biopsy instrument can be inserted through a channel of a flexible endoscope. As the motion transferring member extends within the sleeve, the sleeve has a larger diameter than the motion transferring member. The fact that the sleeve and the motion transferring member have different diameters causes the length relationship between the sleeve and the motion transferring member to be altered as the endoscopic biopsy instrument is bent. If the maximum length that the rotatable cutting tip extends from the distal portion of the sleeve is adjusted prior to the endoscopic biopsy instrument being inserted into the body of the patient, this maximum length will, due to the differences in diameter, be altered as the endoscopic biopsy instrument is bent. Further to that, a flexible motion transferring member is somewhat extended when used, e.g. a motion transferring member comprised of a cable may stretch somewhat when used even though the cable is tightly twisted, as the cable is made up of a plurality of strands and thus has internal cavities. Extension of the flexible motion transferring member alters the relationship between the length of the motion transferring member and the sleeve. In some embodiments, the sleeve and the motion transferring member can be made from different materials which are prolonged differently by thermal changes (such as when the instrument is inserted into the body of the patient). Being able to adjust the maximum length that the rotatable cutting tip extends from the distal portion of the sleeve in situ is thus a significant improvement which increases the precision with which a tissue sample can be obtained.

Endoscope is to be understood as any instrument for viewing or inserting tools for any form of manipulation inside of the body of a patient, in any location. An endoscope could for example be a gastroscope, a colonoscope, a bronchoscope, a rhinoscope, an arthroscope or a trocar.

Biopsy is to be understood as any removal of bodily cells or tissues using an instrument.

Motion transferring member is to be understood as any means for transferring a motion from a first location to a second location positioned at a distance from the first location. A motion transferring member could for example comprise a shaft, a flexible shaft, a Bowden cable, a universal joint, a worm drive, a bevel gear or a combination thereof.

Rotatable cutting tip is to be understood as any means adapted for cutting by means of rotation. According to one embodiment, the rotatable cutting tip could comprise a drill having a screw-like configuration with sharpened threads for cutting the tissue of a patient. Alternatively, the screw-like configuration could be adapted to extract tissue which could be cut against a sleeve in which the screw-like drill is positioned. In alternative embodiments, the rotatable cutting tip could be a cutting tip which is sharp in its distal end, such as a trephine blade.

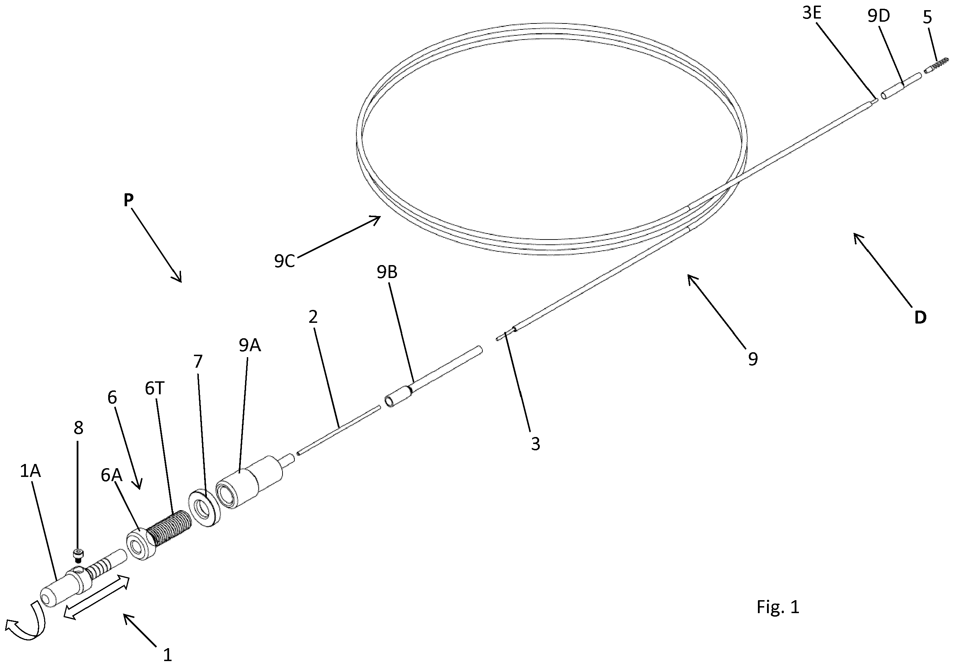

Fig. 1 shows an embodiment of the endoscopic biopsy instrument in an exploded elevated view. The endoscopic biopsy instrument comprises a proximal portion P adapted to remain outside of the body of the patient, when the endoscopic biopsy instrument is in use, and a distal portion D adapted to be inserted into the body of the patient when the endoscopic biopsy instrument is in use.

Starting from the proximal end of the endoscopic biopsy instrument, the instrument comprises a combined element 1 which is an element for manually generating rotary motion and linear motion. The combined element 1 enables the operator to single-handedly operate the biopsy instrument to cut and/or extract tissue using the rotary motion and cut or/and extract the tissue using the linear motion. However, it is equally conceivable that the rotary motion element 1 and the linear motion element 1 are separate elements in alternative embodiments.

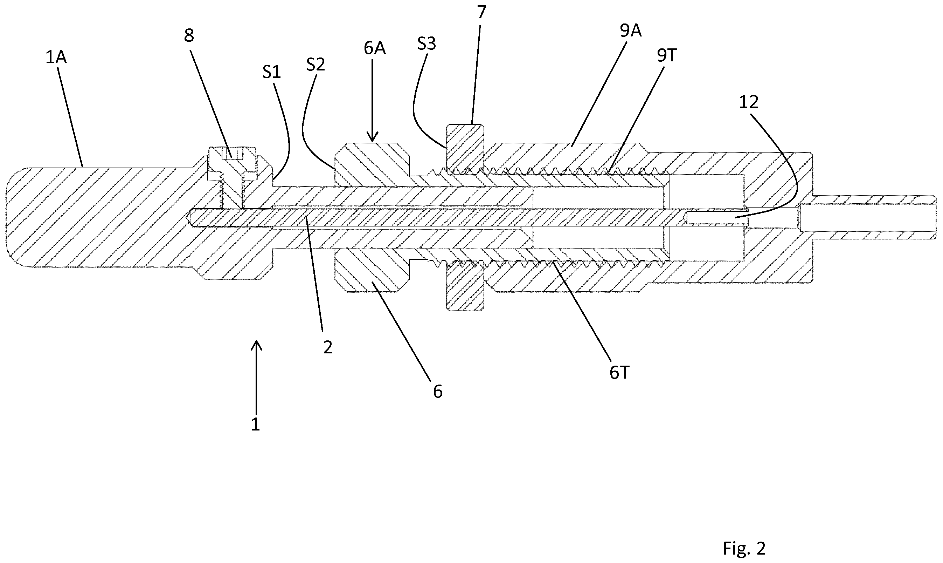

The combined element 1 is in the embodiment shown in fig. 1 connected to a stiff shaft 2 extending from the combined element 1, through the adjustment sleeve 6 and the proximal portion 9A of the sleeve 9, which will be further described with reference to fig. 2. The stiff shaft 2 is in turn connected to a flexible motion transferring member 3 being a flexible shaft 3 comprised of a stranded cable made from a plurality of strands being tightly twisted into a helix. The tightly twisted cable 3 is an important feature in endoscopic biopsy, as it enables the motions performed by the operator at the handle portion to be identically mirrored at the distal portion **D** of the endoscopic biopsy instrument.

The flexible shaft 3 is housed in a sleeve 9 comprising a first, short, rigid portion 9A having a larger diameter, a second rigid portion 9B, having a smaller diameter, a third flexible portion 9C, and a fourth rigid distal portion 9D, through which the rotatable cutting tip 5 extends. The flexible portion 9C of the sleeve 9 could for example comprise a helically wound metal sleeve, a flexible sleeve made from a polymer material, a woven flexible metal sleeve, or a combination thereof.

The distal end 3E of the motion transferring member 3 is fixated to the rotatable cutting tip 5. In the embodiment of fig. 1, the rotatable cutting tip 5 is a screw-like drill with sharpened threads which drills into, and cuts, bodily matter inside the body of the patient. The fitting between the flexible shaft 3 and the rotatable cutting tip 5 could for example be a press fitting, a threaded fitting or a fitting created by means of material bonding.

Turning again to the proximal portion **P** of the endoscopic biopsy instrument, the proximal portion **P** comprises a handle portion which comprises (in addition to the combined element 1) a lockable adjustment device 6, 7, 9A for, in situ, adjusting and locking a maximum length that the rotatable cutting tip 5 extends from the distal portion 9D of the sleeve 9, when being extended and retracted by the combined element 1. The combined element 1 and the stiff shaft 2 extend within the lockable adjustment device 6, 7, 9A, which is further described with reference to fig. 2.

The lockable adjustment device 6, 7, 9A shown in fig. 1 comprises an adjustment sleeve 6, being axially displaceable in relation to the proximal portion 9A of the sleeve 9. The proximal portion 9A of the sleeve 9 comprises an internally threaded portion (shown in section in fig. 2) comprising threads (9T of fig. 2) being adapted to engage the threads 6T of the adjustment sleeve 6, such that the adjustment sleeve 6 can be axially displaced in relation to the sleeve 9, by the adjustment sleeve 6 and the sleeve 9 being radially rotated in relation to each other. The lockable adjustment device 6, 7, 9A further comprises a locking device 7 in the form of a threaded locking member 7 adapted to engage the threads 6T of the adjustment sleeve 6, and being adapted to be screwed fixedly against the proximal portion 9A, for axially locking the adjustment sleeve 6 in relation to the sleeve 9A and thereby adjusting and locking the maximum length that the rotatable cutting tip 5 extends from the distal portion 9D of the sleeve 9, when being extended and retracted by the linear motion element 1.

In alternative embodiments, the proximal portion of sleeve 9A comprises outer threads engaging inner threads of the adjustment sleeve and the locking device, such that the adjustment device and the locking member are axially displaced on the outside of the proximal portion 9A of the sleeve 9.

At least one of the combined element 1 and the lockable adjustment device 6, 7, 9A may comprise grip improving protrusions or recesses (not shown) which may comprise axially extending grooves in particular improving the operators grip when generating rotary motion.

The endoscopic biopsy instrument could for example be used in a channel of an endoscope being a gastroscope or a colonoscope, for obtaining a tissue sample from the digestive system of a patient, or a bronchoscope for obtaining a tissue sample from the airways of a patient, and may have a length exceeding one of 10cm, 20cm, 30cm, 40cm, 50cm, 60cm, 70cm, 80cm, 90cm or 100cm for being able to reach areas of the patient's digestive system or airways.

Fig. 2 shows a detailed sectional view of the handle portion of the proximal portion **P** of the embodiment of the endoscopic biopsy instrument of fig. 1. From the left side of the fig. 2, the combined element 1 is shown, comprising a manipulation portion 1A adapted to be manipulated by the operator. The combined element 1 comprises an internally threaded bore in which a set screw 8 is positioned. The set screw 8 engages a recess of the stiff shaft 2 and thereby axially locks the stiff shaft 2 in the combined element 1. The distal end of the stiff shaft 2 comprises an axially extending bore 12 adapted to receive the proximal end of the flexible shaft 3. The flexible shaft 3 is adapted to be fixated in the axially extending bore 12 by means of press fitting. The combined element 1 runs freely inside of the adjustment sleeve 6, and the proximal portion of the sleeve 9A and linear and rotary motion of the combined element 1, generated by the operator, thus causes extension, retraction and rotation of the rotatable cutting tip 5 at the distal portion **D.**

In the embodiment shown in figs. 1 and 2, the endoscopic biopsy instrument is configured for stepless operation of the extension and retraction of the rotatable cutting tip (5 of fig. 1), and of the rotary motion of the cutting tip. Stepless operation makes it easier to obtain a grip in a hard tumor or bone, as the rotatable cutting tip can be rotated without linear displacement. Stepless operation also enables obtained tissue to be further transported along the threads of the rotatable cutting tip and further into the distal portion of the sleeve (9D of fig. 1) and enables the rotatable cutting tip to be rotated at the same time as it is retracted into the sleeve. The endoscopic biopsy instrument can also be used to pass through tissue at a first area to obtain tissue at a second area, or to obtain tissue from a central location of a larger tissue portion.

The lockable adjustment device 6, 7, 9A will now be further described. The lockable adjustment device 6, 7, 9A of the embodiment of fig. 1 and 2 comprises an adjustment sleeve 6 comprising a manipulation portion 6A, adapted to be rotated by the operator, and a treaded portion 6T, engaging internal threads 9T of the proximal portion of the sleeve 9A, such that rotation of the adjustment sleeve 6 causes axial displacement of the adjustment sleeve in relation to the sleeve 9. The lockable adjustment device 6, 7, 9A further comprises a locking device 7 for locking the lockable adjustment device. The locking device 7 is adapted to be rotated by the operator and comprises internal threads 7T engaging the threads 6T of the adjustment sleeve 6, such that rotation of the locking device 7 causes axial displacement of the locking device 7 along the threaded portion 6T of the adjustment sleeve 6. When the locking device 7 is screwed tightly against the surface S3 of the proximal end of the sleeve 9, the locking ring 7 locks the adjustment sleeve 9 in relation to the sleeve 9 and thus locks the maximum length that the rotatable cutting tip (5 of fig. 1) extends from the distal portion (9D of fig. 1) of the sleeve 9, when the rotatable cutting tip is extended and retracted by the combined element 1.

The combined element shown in fig. 1 has an innermost position, in which the surface S1 of the manipulation portion 1A of the combined element 1 engages the distal surface S2 of the adjustment sleeve 6. When the combined element is in its innermost position, the cutting tip extends maximally from the distal end of the sleeve. The adjustment of the lockable adjustment device adjusts the distance between the proximal end of the sleeve 9 and the innermost position and thus adjusts the maximum length that the rotatable cutting tip (5 of fig. 1) extends from the distal portion (9D of fig. 1), when being extended and retracted by the combined element 1.

In the embodiment shown in figs. 1 and 2, the lockable adjustment device 6, 7, 9A can steplessly adjust the maximum length that the rotatable cutting tip extends from the sleeve, however, in alternative embodiments, the adjustment could be performed in steps.

In alternative embodiments, the lockable adjustment device may comprise a locking device comprising a radially displaceable part adapted to engage at least one of: the motion transferring member, the sleeve and the adjustment sleeve, for locking the maximum length that the rotatable cutting tip extends from the sleeve. The radially displaceable part could lock against the motion transferring member, the sleeve, or the adjustment sleeve by means of friction, or by engaging recesses of the sleeve or adjustment sleeve. The radially displaceable part could be a spring loaded part similar to the spring loaded part of a cord lock for apparel. The radially displaceable part may be adapted to release when pressed, such that the operator can press the radially displaceable part, adjust the a the lockable adjustment device, and release the radially displaceable part for locking the lockable adjustment device.

In a further alternative configuration, the radially displaceable part comprises a silicone disc having a single straight slit or a Y or + shaped slit. When the silicone disc is radially compressed, the slit is opened which enables the motion transferring member, the sleeve or the adjustment sleeve to be displaced in relation to the silicone disc. When the radial pressure is released, the edges of the slit engage the motion transferring member, the sleeve or the adjustment sleeve, locking the lockable adjustment device.

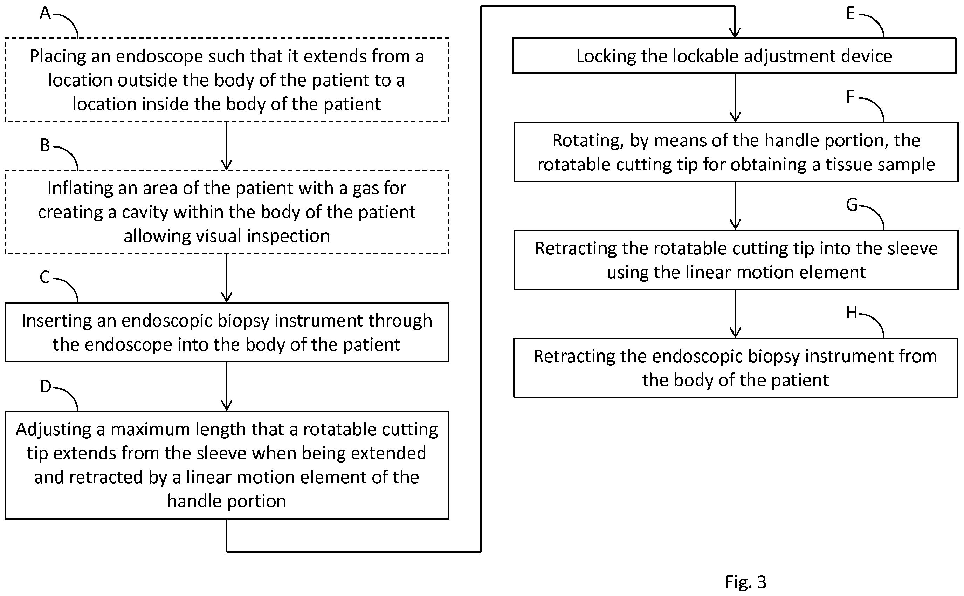

Fig. 3 is a flow chart of a method of using the endoscopic biopsy instrument of figs. 1 and 2 for obtaining a tissue sample from the body of a patient. The method comprises the steps of **C** inserting the endoscopic biopsy instrument through an endoscope into the body of the patient, **D** adjusting a maximum length that the rotatable cutting tip extends from the sleeve when being extended and retracted by a linear motion element of the handle portion, using a lockable adjustment device of the endoscopic biopsy instrument, **E**locking the lockable adjustment device, **F** rotating, by means of the handle portion, the rotatable cutting tip for obtaining a tissue sample, **G** retracting the rotatable cutting tip into the sleeve using the linear motion element, and **H** retracting the endoscopic biopsy instrument from the body of the patient.

The method may be preceded by the steps of **A** placing the endoscope such that it extends from a location outside the body of the patient to a location inside the body of the patient and **B** inflating an area of the patient with a gas for creating a cavity within the body of the patient allowing visual inspection.

The endoscope may comprise a camera for visual inspection, such that the step of **D** adjusting a maximum length that the rotatable cutting tip extends from the sleeve can be performed under visual inspection.

The endoscope may be a gastroscope, and the step of **C** inserting the endoscopic biopsy instrument may comprise inserting the endoscopic biopsy instrument through the gastroscope into the digestive system of the patient, and the step of **F** obtaining a tissue sample may comprise obtaining a tissue sample from the digestive system of the patient.

Alternatively, the endoscope may be a colonoscope, and the step of **C** inserting the endoscopic biopsy instrument through an endoscope into the body of the patient may comprise inserting the endoscopic biopsy instrument through the colonoscope into the digestive system of the patient, and the step of **F** obtaining a tissue sample comprises obtaining a tissue sample from the digestive system of the patient.

Alternatively, the endoscope may be a bronchoscope, and the step of **C** inserting the endoscopic biopsy instrument through an endoscope into the body of the patient may comprise inserting the endoscopic biopsy instrument through the bronchoscope into the airways of the patient, and the step of **F** obtaining a tissue sample may comprise obtaining a tissue sample from the airways of the patient.

Alternatively, the endoscope may be a rhinoscope, and the step of **C** inserting the endoscopic biopsy instrument through an endoscope into the body of the patient may comprise inserting the endoscopic biopsy instrument through the rhinoscope into the nose of the patient, and the step of **F** obtaining a tissue sample may comprise obtaining a tissue sample from the nose of the patient.

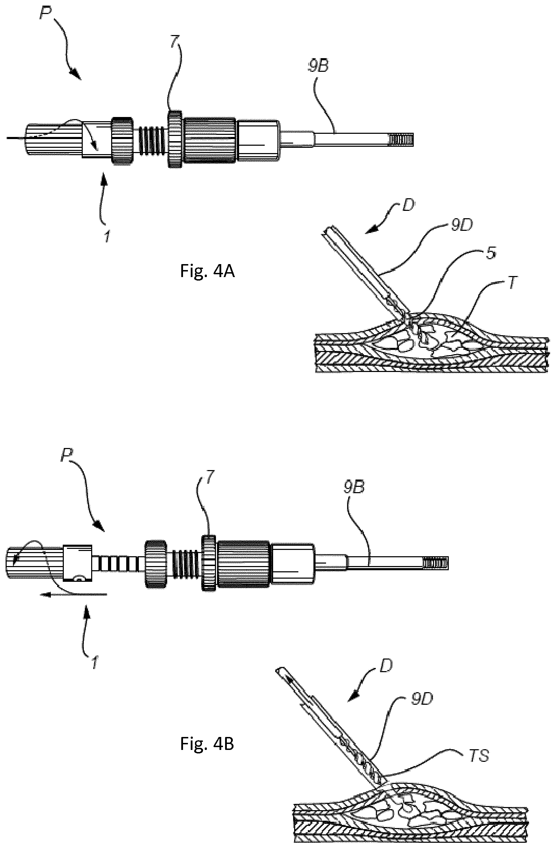

Fig. 4A shows a side view of the proximal portion **P** of the endoscopic biopsy instrument and a sectional view of the distal portion **D** of the endoscopic biopsy instrument when a tissue sample (**TS** in fig 4B) is being obtained. The distal portion **D** has been inserted into the body of the patient through for example an endoscopic biopsy instrument. By means of the combined element 1 and the locking device 7 (such as explained in further detail with reference to fig. 2), the maximum length that the rotatable cutting tip 5 extends from the distal portion of the sleeve 9D is being set in situ. The tissue sample (**TS** of fig. 4B) is being collected by the rotation of the combined element 1, causing the cutting tip 5 to rotate and "drill" into the tissue **T** of the patient until the maximum extension from the sleeve is reached.

Fig. 4B shows the same view of the endoscopic biopsy instrument as in fig. 4A but when the tissue sample **TS** is being retracted into the distal portion of the sleeve 9D. The combined element 1 is being simultaneously retracted and rotated clockwise, such that the cutting tip 5 continuously exerts a retracting force on the tissue sample **TS,** such that the obtained tissue sample **TS** is severed from the rest of the tissue and remains in the distal part of the sleeve 9D as the distal portion **D** of the endoscopic biopsy instrument is being retracted from the body of the patient.

The different aspects or part of the aspects of the disclosed embodiments may all be combined in any possible way, unless clearly contradictory. Any method or any step of a method should be seen also as a description of the elements necessary to carry out said method. Any detailed description should be interpreted in its broadest outline as a general description of the inventive idea.

## Claims

1. An endoscopic biopsy instrument for obtaining a tissue sample from a body of a patient, the endoscopic biopsy instrument comprising:
- a proximal portion (P) adapted to remain outside of the body of the patient in use, and
- a distal portion (D) adapted to be inserted into the body of the patient in use,
- a sleeve (9) extending from the proximal portion to the distal portion, and
- a motion transferring member (3) extending from the proximal portion to the distal portion within the sleeve, **characterized in that** the motion transferring member is configured to transfer rotary and linear motion,
wherein the distal portion comprises a distal end (3E) of the motion transferring member adapted to be connected to a rotatable cutting tip (5) being extendable from a distal end of the sleeve, and being configured to obtain a tissue sample from within the body of the patient using rotary motion, and
wherein the proximal portion comprises a handle portion connected to the sleeve, the handle portion comprising:
- a rotary motion element (1) connected to the motion transferring member for transferring rotary motion to the rotatable cutting tip,
- a linear motion element (1) connected to the cutting tip via the motion transferring member, for extending and retracting the cutting tip from the distal portion of the sleeve, and
- a lockable adjustment device (6, 7, 9A) for, in situ, adjusting and locking a maximum length that the rotatable cutting tip extends from the distal portion of the sleeve, when being extended and retracted by the linear motion element and being rotatable by the rotary motion element (1).

2. The endoscopic biopsy instrument according to claim 1, wherein the linear motion element has an innermost position, in which the cutting tip extends maximally from the distal end of the sleeve, and wherein the lockable adjustment device is adapted to adjust the distance between a proximal end of the sleeve and the innermost position.

3. The endoscopic biopsy instrument according to any one of the preceding claims, wherein the lockable adjustment device comprises an adjustment sleeve (6) being axially displaceable in relation to the sleeve, and wherein the lockable adjustment device further comprises a locking device (7) for axially locking the adjustment sleeve in relation to the sleeve and thereby adjusting and locking the maximum length that the rotatable cutting tip extends from the sleeve, when being extended and retracted by the linear motion element.

4. The endoscopic biopsy instrument according to claim 3, wherein a proximal portion of the sleeve comprises a threaded portion (9T), and wherein the adjustment sleeve comprises threads (6T) being adapted to engage the threads of the sleeve, such that the adjustment sleeve can be axially displaced in relation to the sleeve by the adjustment sleeve and the sleeve being radially rotated in relation to each other.

5. The endoscopic biopsy instrument according to claim 4, wherein the locking device comprises a threaded locking member (7) adapted to engage the threads of at least one of the sleeve and the adjustment sleeve, for locking the sleeve in relation to the adjustment sleeve.

6. The endoscopic biopsy instrument according to any one of the preceding claims, wherein the lockable adjustment device comprises a locking device comprising a radially displaceable part adapted to engage at least one of the motion transferring member, the sleeve and the adjustment sleeve, for locking the maximum length that the rotatable cutting tip extends from the sleeve, when being extended and retracted by the linear motion element.

7. The endoscopic biopsy instrument according to any one of the preceding claims, wherein the rotary motion element and the linear motion element is a combined element (1), such that rotary and linear motion can be generated single-handedly.

8. The endoscopic biopsy instrument according to any one of the preceding claims, wherein the endoscopic biopsy instrument is configured for stepless operation of at least one of:
- the adjustment of the maximum length that the rotatable cutting tip extends from the sleeve,
- the extension and retraction of the rotatable cutting tip, and
- the rotary motion of the cutting tip.

9. The endoscopic biopsy instrument according to any one of the preceding claims, wherein at least one of:
- the sleeve is a flexible sleeve (9C), and
- the motion transferring member is a flexible motion transferring member (3).

10. The endoscopic biopsy instrument according to claim 9, wherein the flexible motion transferring member is a flexible shaft (3).

11. The endoscopic biopsy instrument according to any one of the preceding claims, wherein the motion transferring member has a length exceeding 30cm.

12. The endoscopic biopsy instrument according to claim 3, wherein the linear motion element extends partially inside of the adjustment sleeve and the adjustment sleeve extends partially inside of the sleeve.

13. The endoscopic biopsy instrument according to claims 6, wherein the adjustment sleeve is a compressible adjustment sleeve comprising the radially displaceable part, and wherein:
- compression of the compressible adjustment sleeve disengages the radially displaceable part from the motion transferring member the sleeve or the adjustment sleeve, such that the maximum length that the rotatable cutting tip extends from the distal portion of the sleeve can be adjusted, and
- the release of the compression of the compressible adjustment sleeve engages the radially displaceable part to the motion transferring member the sleeve or the adjustment sleeve, such that the lockable adjustment device is locked.

14. The endoscopic biopsy instrument according to claim 13, wherein at least one of the radially displaceable part and the compressible adjustment sleeve comprises a polymer material.

## Patentansprüche

1. Endoskopisches Biopsieinstrument zum Abnehmen einer Gewebeprobe von einem Körper eines Patienten, wobei das endoskopische Biopsieinstrument Folgendes umfasst:
- einen proximalen Teil (P), der geeignet ist, bei der Verwendung außerhalb des Körpers des Patienten zu bleiben, und
- einen distalen Teil (D), der geeignet ist, bei der Verwendung in den Körper des Patienten eingeführt zu werden,
- einen Schlauch (9), der sich von dem proximalen Teil zum distalen Teil erstreckt, und
- ein bewegungsübertragendes Element (3), das sich von dem proximalen Teil zu dem distalen Teil innerhalb des Schlauchs erstreckt, **dadurch gekennzeichnet, dass** das bewegungsübertragende Element zum Übertragen von Dreh- und linearer Bewegung konfiguriert ist,
wobei der distale Teil ein distales Ende (3 E) des bewegungsübertragenden Elements umfasst, das geeignet ist, mit einer drehbaren Schneidespitze (5) verbunden zu werden, die von einem distalen Ende des Schlauchs ausziehbar und konfiguriert ist, um eine Gewebeprobe unter Anwendung von Drehbewegung von innerhalb des Körpers des Patienten abzunehmen und wobei der proximale Teil einen Griffteil umfasst, der mit dem Schlauch verbunden ist, wobei der Griffteil Folgendes umfasst:
- ein Drehbewegungselement (1), das mit dem bewegungsübertragenden Element zum Übertragen von Drehbewegung auf die drehbare Schneidespitze verbunden ist,
- ein lineares Bewegungselement (1), das mit der Schneidespitze über das bewegungsübertragende Element zum Ausziehen und Zurückziehen der Schneidespitze aus dem distalen Teil des Schlauchs verbunden ist und
- eine absperrbare Einstellungsvorrichtung (6, 7, 9A) zum Einstellen und Absperren, in situ, einer maximalen Länge, über die die drehbare Schneidespitze sich aus dem distalen Teil des Schlauchs erstreckt wenn sie durch das lineare Bewegungselement ausgezogen und zurückgezogen wird, und die durch das Drehbewegungselement (1) drehbar ist.

2. Endoskopisches Biopsieinstrument nach Anspruch 1, wobei das lineare Bewegungselement eine innerste Position aufweist, in der die Schneidespitze sich maximal von dem distalen Ende des Schlauchs aus erstreckt und wobei die absperrenbare Einstellungsvorrichtung geeignet ist, die Entfernung zwischen einem proximalen Ende des Schlauchs und der innersten Position einzustellen.

3. Endoskopisches Biopsieinstrument nach irgendeinem der vorhergehenden Ansprüche, wobei die absperrenbare Einstellungsvorrichtung eine Einstellungsmanschette (6) umfasst, die axial mit Bezug auf den Schlauch verschiebbar ist, und wobei die absperrenbare Einstellungsvorrichtung ferner eine Absperrungsvorrichtung (7) zum axialen Absperren der Einstellungsmanschette mit Bezug auf den Schlauch und dadurch Einstellen und Absperren der maximalen Länge, über die die drehbare Schneidespitze sich von dem Schlauch aus erstreckt, wenn sie durch das lineare Bewegungselement ausgezogen und zurückgezogen wird, umfasst.

4. Endoskopisches Biopsieinstrument nach Anspruch 3, wobei ein proximaler Teil des Schlauchs einen Gewindeteil (9T) umfasst und wobei die Einstellungsmanschette Gewinde (6T) umfasst, die geeignet sind, die Gewinde des Schlauchs einzurasten, derart, dass die Einstellungsmanschette axial mit Bezug auf den Schlauch dadurch verschoben werden kann, dass die Einstellungsmanschette und der Schlauch radial mit Bezug aufeinander gedreht werden.

5. Endoskopisches Biopsieinstrument nach Anspruch 4, wobei die Absperrungsvorrichtung ein Gewindeabsperrungselement (7) umfasst, das geeignet ist, die Gewinde von mindestens einem von dem Schlauch und der Einstellungsmanschette zum Absperren des Schlauchs mit Bezug auf die Einstellungsmanschette einzurasten.

6. Endoskopisches Biopsieinstrument nach irgendeinem der vorhergehenden Ansprüche, wobei die absperrfähige Einstellungsvorrichtung eine Absperrungsvorrichtung umfasst, die ein radial verschiebbares Teil umfasst, das zum Einrasten mindestens eines von dem bewegungsübertragenden Element, dem Schlauch und der Einstellungsmanschette zum Absperren der maximalen Länge, über die die drehbare Schneidelippe sich von dem Schlauch aus erstreckt, geeignet ist, wenn sie durch das lineare Bewegungselement ausgezogen und zurückgezogen wird.

7. Endoskopisches Biopsieinstrument nach irgendeinem der vorhergehenden Ansprüche, wobei das Drehbewegungselement und das lineare Bewegungselement ein kombiniertes Element (1) sind, derart, dass die Dreh- und lineare Bewegung einhändig erzeugt werden können.

8. Endoskopisches Biopsieinstrument nach irgend einem der vorhergehenden Ansprüche, wobei das endoskopische Biopsieinstrument zum stufenlosen Ausführen mindestens eines konfiguriert ist von:
- der Einstellung der maximalen Länge, über die die drehbare Schneidespitze sich von dem Schlauch aus erstreckt,
- dem Ausziehen und Zurückziehen der drehbaren Schneidespitze und
- der Drehbewegung der Schneidespitze.

9. Endoskopisches Biopsieinstrument nach irgendeinem der vorhergehenden Ansprüche, wobei mindestens eines von:
- dem Schlauch ein flexibler Schlauch (9C) ist und
- das Bewegungsübertragungselement ein flexibles Bewegungsübertragungselement (3) ist.

10. Endoskopisches Biopsieinstrument nach Anspruch 9, wobei das flexible Bewegungsübertragungselement eine flexible Welle (3) ist.

11. Endoskopisches Biopsieinstrument nach irgendeinem der vorhergehenden Ansprüche, wobei das Bewegungsübertragungselement eine Länge aufweist, die 30 cm übersteigt.

12. Endoskopisches Biopsieinstrument nach Anspruch 3, wobei das lineare Bewegungselement sich teilweise innerhalb der Einstellungsmanschette erstreckt und die Einstellungsmanschette sich teilweise innerhalb des Schlauchs erstreckt.

13. Endoskopisches Biopsieinstrument nach Anspruch 6, wobei die Einstellungsmanschette eine komprimierbare Einstellungsmanschette ist, die den radial verschiebbaren Teil umfasst, und wobei:
- die Kompression der komprimierbaren Einstellungsmanschette das radial verschiebbare Teil von dem Bewegungsübertragungselement, dem Schlauch oder der Einstellungsmanschette löst, derart, dass die maximale Länge, über die die drehbare Schneidelippe sich von dem distalen Teil des Schlauchs aus erstreckt, eingestellt werden kann, und
- das Auslösen der Kompression der komprimierbaren Einstellungsmanschette das radial verschiebbare Teil in das bewegungsübertragende Element, den Schlauch oder die Einstellungsmanschette einrastet, derart, dass die absperrbare Einstellungsvorrichtung abgesperrt wird.

14. Endoskopisches Biopsieinstrument nach Anspruch 13, wobei mindestens eines von dem radial verschiebbaren Teil und der komprimierbaren Einstellungsmanschette ein Polymermaterial umfasst.

## Revendications

1. Instrument de biopsie endoscopique pour obtenir un échantillon de tissu d'un corps d'un patient, l'instrument de biopsie endoscopique comprenant:
- une partie proximale (P) adaptée pour rester à l'extérieur du corps du patient lors de l'utilisation, et
- une partie distale (D) adaptée pour être insérée dans le corps du patient lors de l'utilisation,
- une chemise (9) s'étendant de la partie proximale à la partie distale, et
- un élément de transfert de mouvement (3) s'étendant de la partie proximale à la partie distale dans la chemise,
**caractérisé en ce que** l'élément de transfert de mouvement est configuré pour transférer un mouvement rotatif et linéaire,
où la partie distale comprend une extrémité distale (3E) de l'élément de transfert de mouvement adaptée pour être connectée à une pointe de découpage rotative (5) qui est extensible à partir d'une extrémité distale de la chemise, et étant configurée pour obtenir un échantillon de tissu de l'intérieur du corps du patient en utilisant un mouvement rotatif, et
où la partie proximale comprend une partie de poignée connectée à la chemise, la partie de poignée comprenant:
- un élément de mouvement rotatif (1) connecté à l'élément de transfert de mouvement pour transférer le mouvement rotatif à la pointe de découpage rotative,
- un élément de mouvement linéaire (1) connecté à la pointe de découpage via l'élément de transfert de mouvement, pour étendre et rétracter la pointe de découpage de la partie distale de la chemise, et un dispositif d'ajustement verrouillable (6, 7, 9A) pour, in situ, ajuster et verrouiller une longueur maximum où la pointe de découpage rotative s'étend de la partie distale de la chemise, quand elle est étendue et rétractée par l'élément de mouvement linéaire et pouvant être rotative par l'élément de mouvement rotatif (1).

2. Instrument de biopsie endoscopique selon la revendication 1, dans lequel l'élément de mouvement linéaire a une position la plus interne, dans laquelle la pointe de découpage s'étend maximalement de l'extrémité distale de la chemise, et où le dispositif d'ajustement verrouillable est adapté pour ajuster la distance entre une extrémité proximale de la chemise et la position la plus interne.

3. Instrument de biopsie endoscopique selon l'une quelconque des revendications précédentes, dans lequel le dispositif d'ajustement verrouillable comprend comprend une chemise d'ajustement (6) qui est déplaçable axialement relativement à la chemise, et où le dispositif d'ajustement verrouillable comprend en outre un dispositif de verrouillage (7) pour verrouiller axialement la chemise d'ajustement relativement à la chemise et ainsi ajuster et verrouiller la longueur maximum où la pointe de découpage rotative s'étend de la chemise, quand elle est étendue et rétractée par l'élément de mouvement linéaire.

4. Instrument de biopsie endoscopique selon la revendication 3, dans lequel une partie proximale de la chemise comprend une partie filetée (9T) et où la chemise d'ajustement comprend les filetages (6T) qui sont adaptés pour mettre en prise les filetages de la chemise, de telle manière que la chemise d'ajustement peut être déplacée axialement relativement à la chemise et la chemise étant radialement tournée relativement l'une à l'autre.

5. Instrument de biopsie endoscopique selon la revendication 4, dans lequel le dispositif de verrouillage comprend un élément de verrouillage fileté (7) adapté pour mettre en prise les filetages d'au moins une de la chemise et de la chemise d'ajustement, pour verrouiller la chemise relativement à la chemise d'ajustement.

6. Instrument de biopsie endoscopique selon l'une quelconque des revendications précédentes, dans lequel le dispositif d'ajustement verrouillable comprend un dispositif de verrouillage comprenant une partie radialement déplaçable adaptée pour mettre en prise au moins un de l'élément de transfert de mouvement, de la chemise et de la chemise d'ajustement, pour verrouiller la longueur maximum où la pointe de découpage rotative s'étend de la chemise, quand elle est étendue et rétractée par l'élément de mouvement linéaire.

7. Instrument de biopsie endoscopique selon l'une quelconque des revendications précédentes, dans lequel l'élément de mouvement rotatif et l'élément de mouvement linéaire est un élément combiné (1), de telle manière qu'un mouvement rotatif et linéaire peut être produit tout seul.

8. Instrument de biopsie endoscopique selon l'une quelconque des revendications précédentes, dans lequel l'instrument de biopsie endoscopique est configuré pour une opération sans étapes d'au moins un de:
- l'ajustement de la longueur maximum où la pointe de découpage rotative s'étend de la chemise,
- l'extension et la rotation de la pointe de découpage rotative, et
- le mouvement rotatif de la pointe de découpage.

9. Instrument de biopsie endoscopique selon l'une quelconque des revendications précédentes, dans lequel au moins un de:
- la chemise est une chemise flexible (9C), et
- l'élément de transfert de mouvement est un élément de transfert de mouvement flexible (3).

10. Instrument de biopsie endoscopique selon la revendication 9, dans lequel l'élément de transfert de mouvement flexible est un arbre flexible (3).

11. Instrument de biopsie endoscopique selon l'une quelconque des revendications précédentes, dans lequel l'élément de transfert de mouvement a une longueur dépassant 30 cm.

12. Instrument de biopsie endoscopique selon la revendication 3, dans lequel l'élément de mouvement linéaire s'étend partiellement à l'intérieur de la chemise d'ajustement et la chemise d'ajustement s'étend partiellement à l'intérieur de la chemise.

13. Instrument de biopsie endoscopique selon la revendication 6, dans lequel la chemise d'ajustement est une chemise d'ajustement compressible comprenant la partie déplaçable radialement, et où:
- la compression de la chemise d'ajustement compressible désengage la partie déplaçable radialement de l'élément de transfert de mouvement la chemise ou la chemise d'ajustement, de telle manière que la longueur maximum où la pointe de découpage rotative s'étend de la partie distale de la chemise peut être ajustée, et
- la libération de la compression de la chemise d'ajustement compressible met en prise la partie déplaçable radialement à l'élément de transfert de mouvement la chemise ou l'ajustement de chemise, de telle manière que le dispositif d'ajustement verrouillable est verrouillé.

14. Instrument de biopsie endoscopique selon la revendication 13, dans lequel au moins une de la partie déplaçable radialement et de la chemise d'ajustement compressible comprend un matériau polymère.
